(19) Europäisches Patentamt / European Patent Office / Office européen des brevets

(11) **EP 4 545 976 A1**

(12) **EUROPEAN PATENT APPLICATION**
published in accordance with Art. 153(4) EPC

(43) Date of publication:
**30.04.2025 Bulletin 2025/18**

(21) Application number: **23826976.5**

(22) Date of filing: **06.06.2023**

(51) International Patent Classification (IPC):
*G01N 35/02* (2006.01)    *G06T 7/00* (2017.01)
*G06T 7/12* (2017.01)    *G01N 33/48* (2006.01)
*G01N 21/88* (2006.01)

(52) Cooperative Patent Classification (CPC):
**G01N 21/88; G01N 33/48; G01N 35/02; G06T 7/00;
G06T 7/12**

(86) International application number:
**PCT/JP2023/021066**

(87) International publication number:
**WO 2023/248789 (28.12.2023 Gazette 2023/52)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.06.2022 JP 2022101278**

(71) Applicant: HITACHI HIGH-TECH CORPORATION
Tokyo 105-6409 (JP)

(72) Inventors:
• **HATTORI, Hideharu**
  **Tokyo 100-8280 (JP)**
• **KAKISHITA, Yasuki**
  **Tokyo 100-8280 (JP)**
• **TAMEZANE, Hideto**
  **Tokyo 105-6409 (JP)**
• **SUZUKI, Yoichiro**
  **Tokyo 105-6409 (JP)**

(74) Representative: **MERH-IP Matias Erny Reichl
Hoffmann
Patentanwälte PartG mbB
Paul-Heyse-Straße 29
80336 München (DE)**

(54) **SAMPLE CONDITION ASSESSING DEVICE, SAMPLE CONDITION ASSESSING METHOD, AND SAMPLE TESTING DEVICE**

(57) A specimen condition assessing device includes a processor that executes a program for performing image processing on an image of a target specimen container and a memory for storing a result of the image processing. The processor performs processing of inputting the image, processing of detecting a boundary position from the image, processing of determining a type of upper and lower regions of each boundary position and determining a boundary position of each inclusion, and processing of assessing whether or not the target specimen container is testable on the basis of the boundary position of each inclusion.

FIG. 1

EP 4 545 976 A1

**Description**

Incorporation by Reference

The present application claims priority from Japanese Patent Application No. 2022-101278 filed on Jun. 23, 2022, the content of which is hereby incorporated by reference into this application.

Technical Field

The present invention relates to a specimen condition assessing device, a specimen condition assessing method, and a specimen testing device, and relates to an image processing technology using machine learning which is for, e.g., detecting and identifying a specific inclusion in a specimen (such as, e.g., a serum (blood plasma)) included in a photographed image.

Background Art

In recent years, the importance of blood testing has been growing and, in blood analysis also, the number of tests and the number of testing items have increased, and improved testing efficiency is required. To improve the testing efficiency, there is an increasing need to detect a precise content of a serum or the like or assess a testable sample container before testing. To detect a content of serum or the like, there are technologies proposed in, e.g., Japanese Unexamined Patent Application Publication No. Hei10(1998)-232228, Japanese Unexamined Patent Application Publication No. Hei09(1997)-133687, and Japanese Unexamined Patent Application Publication No. 2005-265813. In Japanese Unexamined Patent Application Publication No. Hei10(1998)-232228, a plurality of color extraction means are used to detect upper and lower boundary positions of a serum. In Japanese Unexamined Patent Application Publication No. Hei09(1997)-133687, saturation information is used to determine a boundary position between a serum portion and another portion and calculate an amount of the serum. In Japanese Unexamined Patent Application Publication No. 2005-265813, a mechanism that emits and receives an infrared ray is vertically moved to detect respective positions of a separating agent, a serum, and a blood clot.

Summary of Invention

Technical Problem

However, air bubbles may occasionally be generated on a sample to result in a problem that, even when the plurality of color extraction means in Japanese Unexamined Patent Application Publication No. Hei10(1998)-232228, the saturation information in Japanese Unexamined Patent Application Publication No. Hei09(1997)-133687, and the infrared ray emission/reception mechanism in Japanese Unexamined Patent Application Publication No. 2005-265813 are used, influence of the air bubbles cannot be suppressed and the precise boundary positions of the serum or the like cannot be determined. In addition, in each of Japanese Unexamined Patent Application Publication No. Hei10(1998)-232228, Japanese Unexamined Patent Application Publication No. Hei09(1997)-133687, and Japanese Unexamined Patent Application Publication No. 2005-265813, a problem is encountered in that contents of all the inclusions such as the serum, the separating agent, and the blood clot cannot be determined or whether or not a target sample container is testable cannot be determined for a sample container containing a sample including a combination of these inclusions.

The present invention has been achieved in view of such circumstances and provides a technology that allows boundary positions of a serum (blood plasma) or the like in an image to be detected and allows whether or not a specimen container is testable to be precisely assessed.

Solution to Problem

A specimen condition assessing device according to an aspect of the present invention includes a processor that executes a program for performing image processing on an image of a target specimen container and a memory for storing a result of the image processing. The processor performs processing of inputting the image, processing of detecting one or more boundary position from the image, processing of determining a property (type) of upper and lower regions of each of the one or more boundary positions and determining a boundary position of each inclusion, and processing of assessing whether or not the target specimen container is testable on the basis of the determined boundary position of each inclusion.

Further features related to the present invention will be made apparent from the statement in this description and the accompanying drawings. The aspect of the present invention is attained and implemented by elements, a combination of various elements, the following detailed statement, and a form of the appended claims.

It is necessary to understand that the statement in this description is merely a typical illustration and by no means limits the claims or application examples of the present invention.

Advantageous Effects of Invention

According to the aspect of the present invention, it is possible to detect a boundary position in an image and precisely assess whether or not a specimen container is testable.

Brief Description of Drawings

Fig. 1 is a block diagram illustrating functions of a specimen condition assessing device according to a first embodiment.
Fig. 2 is a diagram illustrating an example of a hardware configuration of the specimen condition assessing device according to the first embodiment.
Fig. 3 is a diagram for illustrating an example of an operation of a container identifying unit.
Fig. 4 is a diagram for illustrating an example of an operation of a boundary position detecting unit.
Fig. 5 is a diagram for illustrating an example of an operation of an inclusion determining unit.
Fig. 6 is a diagram for illustrating an example of an operation of a boundary position correcting unit.
Fig. 7 is a diagram for illustrating an example of an operation of a content calculating unit.
Fig. 8 is a diagram for illustrating an example of the operation of the inclusion determining unit.
Fig. 9 is a diagram for illustrating an example of identification of upper and lower regions of each boundary by the inclusion determining unit.
Fig. 10 is a diagram for illustrating an example of identification of each boundary by the boundary position correcting unit.
Fig. 11 is a diagram for illustrating an example of identification of air bubbles by the boundary position correcting unit.
Fig. 12 is a diagram for illustrating an example of an operation of the content calculating unit.
Fig. 13 is a diagram for illustrating an example of an operation of a sample condition assessing unit.
Fig. 14 is a diagram for illustrating an example of display of a determination result or the like from an output unit.
Fig. 15 is a flow chart for illustrating an overall operation of a specimen condition assessing device 1 according to the first embodiment.
Fig. 16 is a diagram illustrating a schematic configuration of a sample testing device according to a second embodiment in which the specimen condition assessing device in the embodiment is mounted.

Description of Embodiments

An embodiment of the present description provides a specimen condition assessing device and a specimen condition assessing method that detect boundary positions of a plurality of inclusions, while determining a type (property) of each of the inclusions around each of the boundary positions, further calculate a content thereof, while correcting the boundary position, and determine the presence or absence of each of the inclusions (such as, e.g., a serum (blood plasma), a separating agent, a blood clot, and urine) of a sample in a container on the basis of the plurality of boundary positions and a result of the determination of the inclusion to thereby implement high-precision determination of the content of each of the inclusions (such as, e.g., the serum (blood plasma), separating agent, blood clot, and urine) and assessment of whether or not a target sample container is testable.

Referring to the accompanying drawings, a description will be given below of embodiments of the present invention. In the drawings, functionally identical elements may be represented by the same numerals. Note that the accompanying drawings illustrate specific embodiments and implementation examples based on a principle of the present invention, but these are provided to allow the present invention to be understood and should not be used to limitatively construe the present invention.

While sufficiently detailed description for a person skilled in the art to implement the present invention is made in this embodiment, other implementations and modes are also possible, and it is necessary to understand that configurations/structures can be changed and various elements can be replaced without departing from the scope and spirit of the technical idea of the present invention. Therefore, the following description should not be construed as being limited to this.

Furthermore, as will be described later, each of the embodiments of the present invention may also be implemented using software operating on a versatile computer or may also be implemented using dedicated hardware or a combination of software and hardware.

The following will describe each processing in the embodiments of the present invention by using "Each of processing units (such as, e.g., an inclusion determining unit) as a program" as a subject (operating entity) but, since the program performs determined processing while using a memory and a communication port (communication control device) by being executed by a processor (such as a CPU), the description may also be given by using the processor as the subject.

A specimen condition assessing device in one of the embodiments of the present description may also include a function of detecting a boundary position in a container and determining an inclusion and a function of assessing a condition of a specimen container. The specimen container is a combination of a container and a specimen contained in the container. The specimen condition assessing device may further include a function of correcting the boundary position. The embodiment in the present description detects the boundary position even when there is an air bubble or the like in the vicinity of the boundary position, determines the inclusions, the air bubble, and the like around the boundary position, analyzes the likelihood of a boundary around the boundary position in detail, and corrects the boundary position. This allows the boundary position of a serum (blood plasma) or the like in an image and a content thereof to be determined with high precision. By additionally determining the presence or absence of each of inclusions (such as a serum (blood plasma), a separating agent, a blood clot, and urine) in the container on the basis of a plurality of the boundary positions and the result of determining the inclusion, it is possible to assess whether or not the target sample container is testable.

(1) First Embodiment

<Functional Configuration of Specimen Condition Assessing Device>

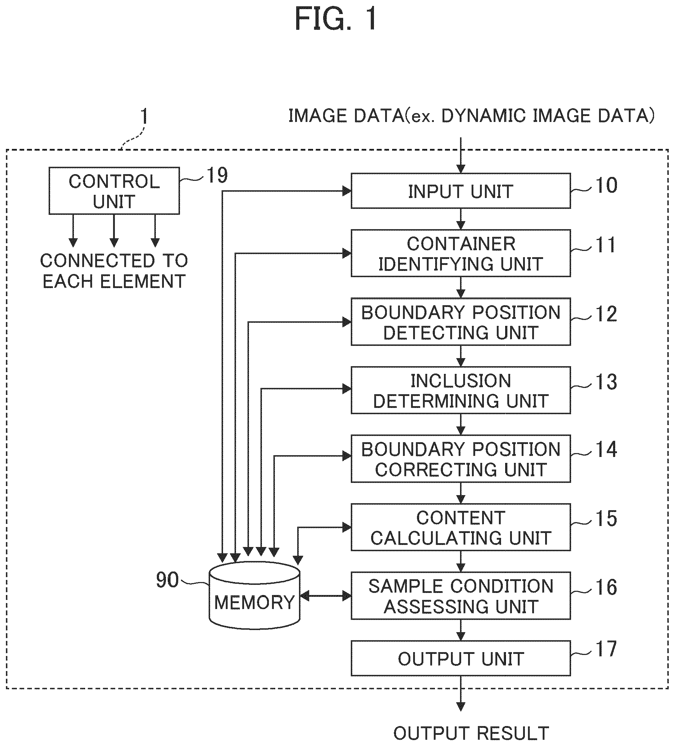

Fig. 1 is a block diagram illustrating a functional configuration of a specimen condition assessing device according to the embodiment. The specimen condition assessing device 1 includes an input unit 10, a container identifying unit 11, a boundary position detecting unit 12, an inclusion determining unit 13, a boundary position correcting unit 14, a content calculating unit 15, a sample condition assessing unit 16, a control unit 19, and a memory 90. Note that, in the specimen condition assessing device, when container information has been given in advance, the container identifying unit 11 is not an essential component. The specimen condition assessing device 1 may also be mounted in a sample testing device, as will be described later (second embodiment). The embodiments described hereinbelow are applicable also to a specimen different from a sample collected from a human.

In the specimen condition assessing device 1, the container identifying unit 11, the boundary position detecting unit 12, the inclusion determining unit 13, the boundary position correcting unit 14, the content calculating unit 15, and the sample condition assessing unit 16 may be implemented by programs or may also be implemented by being modularized.

To the input unit 10, image data is input. For example, the input unit 10 may acquire encoded still image data in a JPG, Jpeg 2000, PNG, BMP, or like format resulting from image capturing performed at predetermined time intervals by an image capturing means, such as a camera built in an image acquiring device, and use an image thereof as an input image. Alternatively, the input unit 10 may also extract the still image data of frames at predetermined intervals from dynamic image data in a Motion JPEG, MPEG, H. 264, HD/SDI, or like format, and use an image thereof as the input image. Still alternatively, the input unit 10 may also use an image acquired by the image capturing means via a bus, a network, or the like as the input image. Yet alternatively, the input unit 10 may also use an image already stored in a detachable recording medium as the input image. The image input from the input unit 10 is output via the memory 90 to the container identifying unit 11, the boundary position detecting unit 12, the inclusion determining unit 13, and the boundary position correcting unit 14.

The container identifying unit 11 uses a network that detects the entire container or a portion of the container (e.g., a container detector such as YOLO) to detect the container from the input image and identify the input image. In addition, the container identifying unit 11 stores the calculated container information in the memory 90.

The boundary position detecting unit 12 uses a network that detects each boundary position and barcode boundaries (e.g., a boundary position detector such as YOLO) to detect each boundary position and the barcode boundaries from the input image. The boundary position is an interface between individual inclusions or between each inclusion and another substance. The barcode represents, e.g., information on a sample or a patient from which the sample has been collected. The boundary position detecting unit 12 stores, in the memory 90, calculated information such as each boundary position and the barcode boundaries.

The inclusion determining unit 13 uses an identifier formed of a network (such as, e.g., Convolutional Neural Network) that identifies inclusions (such as, e.g., a serum (blood plasma), a separating agent, a blood clot, and urine) in upper and lower regions of each boundary position and the barcode each detected by the boundary position detecting unit 12, an object other than the inclusions (such as, e.g., background), and the barcode to calculate an identification result of the upper and lower regions of each boundary position and the barcode. In addition, the inclusion determining unit 13 stores, in the memory 90, information such as the calculated identification result of the upper and lower regions of each boundary position and the barcode.

The boundary position correcting unit 14 uses an identifier formed of a network (such as, e.g., Convolutional Neural Network) that identifies each boundary position in detail to identify a periphery of each boundary position detected by the boundary position detecting unit 12 in detail, detect a precise boundary position, and correct the boundary position. In addition, the boundary position correcting unit 14 uses an identifier formed of a network (such as, e.g., Convolutional Neural Network) that identifies the presence or absence of a bubble around each boundary position to identify the presence or absence of a bubble and correct, when there is a bubble, the boundary position to a lowermost end of the bubble. In addition, the boundary position correcting unit 14 stores, in the memory 90, information such as each corrected boundary position.

The content calculating unit 15 calculates a content of the target inclusion (such as, e.g., the serum (blood plasma), separating agent, blood clot, or urine) in the container from each boundary position corrected by the boundary position correcting unit 14 and container information or container information identified by the container identifying unit 11. In addition, the content calculating unit 15 stores, in the memory 90, information such as the calculated content of each of the inclusions in the container.

The sample condition assessing unit 16 uses the content calculated by the content calculating unit 15 or the identification result of the individual regions obtained by the inclusion determining unit 13 to assess whether or not the target sample container (container containing a sample) is testable. In addition, the sample condition assessing unit 16 stores, in the memory 90, information such as a result of assessing whether or not the target container is testable.

The control unit 19 is implemented by a processor and connected to each of elements in the specimen condition assessing device 1. An operation of each of the elements of the specimen condition assessing device 1 is implemented by an autonomous operation of each of the components described above or according to an instruction from the control unit 19.

Thus, in the specimen condition assessing device 1 in the present embodiment, the container identifying unit 11 identifies a type of the container. The boundary position detecting unit 12 detects each boundary position in the container and the barcode boundary positions. The inclusion determining unit 13 calculates the identification result of the upper and lower regions of each boundary position in the container and the barcode. The boundary position correcting unit 14 identifies the periphery of each boundary position in detail to correct the boundary position, while the content calculating unit 15 calculates the content of the target inclusion in the container. The sample condition assessing unit 16 uses the calculated content and the identification result of the peripheral regions of each boundary position to assess a condition of a sample in the container, thereby detect the content of the inclusion in the container from the image with high precision, and assess whether or not the target container is testable.

<Hardware Configuration of Identifier Generating Device>

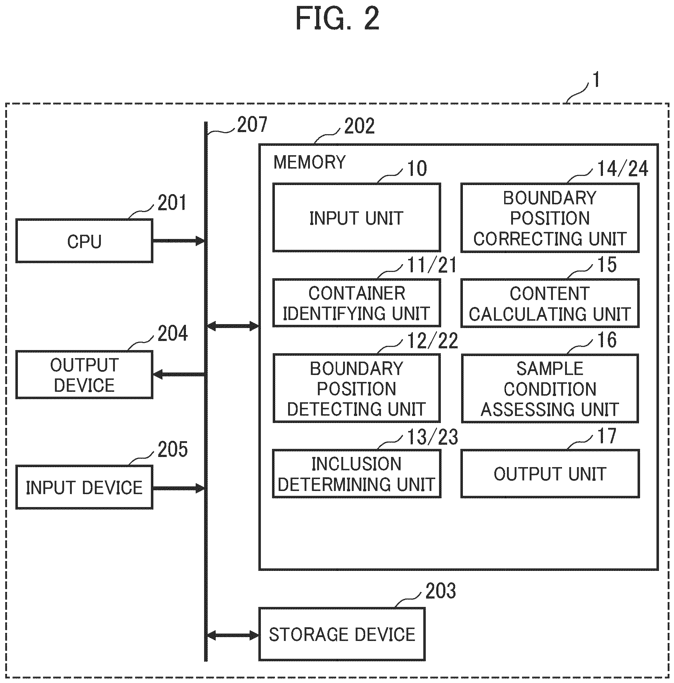

Fig. 2 is a diagram illustrating an example of a hardware configuration of the specimen condition assessing device 1 according to the embodiment of the present invention.

The specimen condition assessing device 1 includes a CPU (processor) 201 that executes various programs, a memory 202 that stores the various programs, a storage device (corresponding to the memory 90) 203 that stores various data, an output device 204 for outputting each boundary position, each content, the result of assessing whether or not the target container is testable, and the like, and an input device 205 for inputting an instruction from a user, an image, and the like, which are connected to each other via a bus 207.

The CPU 201 reads the various programs from the memory 202 as necessary, and executes the programs. The memory 202 stores the input unit 10, the container identifying unit 11, the boundary position detecting unit 12, the inclusion determining unit 13, the boundary position correcting unit 14, the content calculating unit 15, and the sample condition assessing unit 16 each as the program.

The storage device 203 stores the input image, information on the type of the container identified by the container identifying unit 11, information on each boundary position in the container and the barcode boundary positions each detected by the boundary position detecting unit 12, the identification result of the upper and lower regions of each boundary position in the container and the barcode that is determined by the inclusion determining unit 13, information on the boundary position corrected by the boundary position correcting unit 14, information on the content of the target inclusion in the container calculated by the content calculating unit 15, the result of the assessment of whether or not the target container is testable performed by the sample condition assessing unit 16, and the like.

The output device 204 is configured to include devices such as a display, a printer, and a speaker. For example, the output device 204 displays, on a display screen, data generated by the container identifying unit 11, the boundary position detecting unit 12, the inclusion determining unit 13, the boundary position correcting unit 14, the content calculating unit 15, and the sample condition assessing unit 16.

The input device 205 is configured to include devices such as a keyboard, a mouse, and a microphone. By the input device 205, instructions (including determination of the inputting of the input image) from the user are input to the specimen condition assessing device 1.

The specimen condition determining device of the present invention identifies the container type, detects each boundary position in the container and the barcode boundary positions, calculates the identification result of the upper and lower regions of each boundary position in the container and the barcode, identifies the periphery of each boundary position in detail to correct the boundary position, calculates the content of the target inclusion in the container, and further assesses the condition of the sample in the container by using the calculated content and the identification result of the peripheral regions of each boundary position to thereby precisely assessing whether or not the target container is testable, while detecting the content of the inclusion in the container from the image with high precision.

<Configuration and Operation of Each Unit>

Hereinbelow, a detailed description will be given of a configuration and an operation of each of the elements.

(i) Container Identifying Unit 11

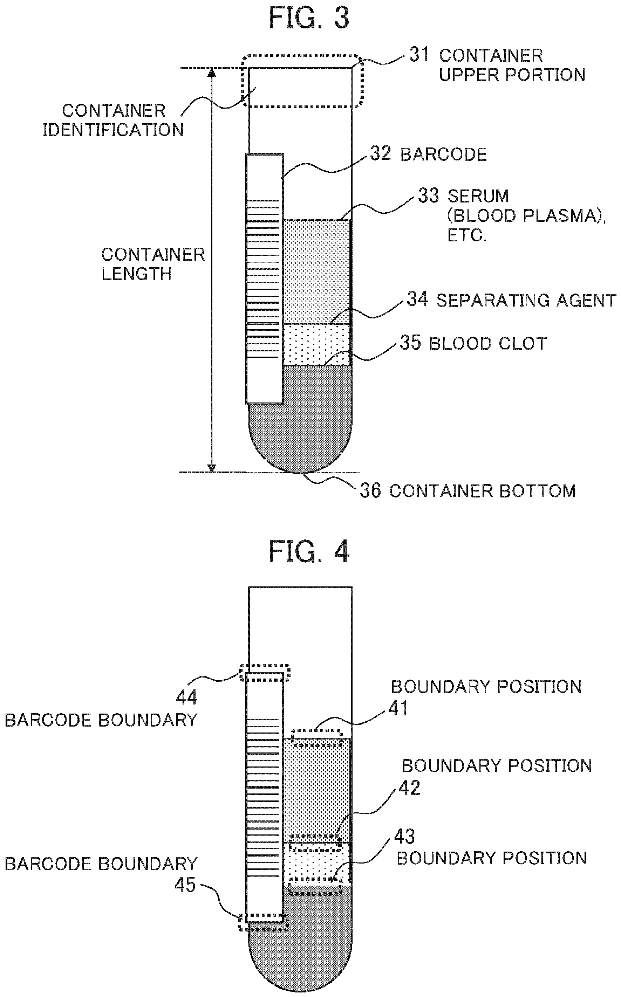

Fig. 3 illustrates an example of the container. The container identifying unit 11 uses a network (a container detector such as, e.g., YOLO) that detects the entire container or a portion of the container to detect and identify the container from the input image, and determine a container type ct and a position of a container upper portion 31, while calculating a position of a container bottom 36 in the image, which is illustrated in Fig. 3, from the calculated container type ct and container information in the memory 90. The container identifying unit 11 stores, in the memory 90, information on the identified container type and the respective positions of the container upper portion and the container bottom in the image.

(ii) Boundary Position Detecting Unit 12

The boundary position detecting unit 12 detects the boundary positions (interfaces or boundaries) of the individual inclusions in the container and the barcode boundaries. Fig. 3 illustrates a barcode 32, a serum 33, a separating agent 34, and a blood clot 35. Fig. 4 illustrates an example of boundary position detection. The boundary position detecting unit 12 uses a network (such as a boundary position detector such as, e.g., YOLO) that detects each of the boundary positions (such as the boundaries of the inclusions) in the container and the barcode boundaries to detect each of the boundary positions and the boundaries of the barcode 32 from the input image as illustrated in Fig. 4. In the example illustrated in Fig. 4, a boundary 41 between the serum (blood plasma) 33 and the background, a boundary 42 between the serum 33 and the separating agent 34, and a boundary 43 between the blood clot and the separating agent are detected. In addition, an upper boundary 44 and a lower boundary 45 of the barcode 32 are detected.

When none of boundaries such as the boundary positions can be detected, the boundary position detecting unit 12 sets 0 to a boundary position detection result lr and stores the boundary position detection result lr in the memory. When detecting one or more boundaries such as the boundary positions, the boundary position detecting unit 12 sets the number of the detected boundaries to the boundary position detection result lr. The boundary position detecting unit 12 stores, in the memory 90, the calculated boundary position detection results lr and information on each of the detected boundary positions.

(iii) Inclusion Determining Unit 13

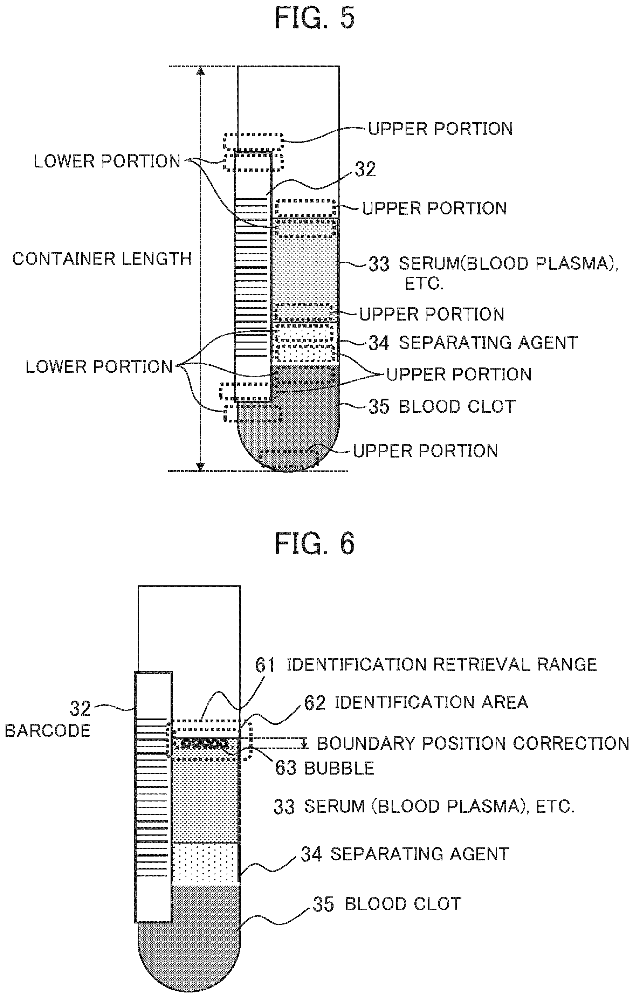

As illustrated in Fig. 5, the inclusion determining unit 13 uses an identifier formed of a network (such as, e.g., Convolutional Neural Network) that identifies inclusions (inclusions such as, e.g., the serum (blood plasma), separating agent, blood clod, and urine) and objects other than the inclusions (such as, e.g., the background and the barcode) to calculate, for the upper and lower regions of each of the boundary positions and the barcode each detected by the boundary position detecting unit 12, the identification result of the upper and lower regions of each boundary position and the barcode. When containers include containers made of various materials such as a transparent-type container and an opaque-type container, the inclusion determining unit 13 uses information on the container type identified by the container identifying unit 11 to select an identifier appropriate for the container, and uses the selected identifier to calculate the identification result of the upper and lower regions of each boundary position and the barcode.

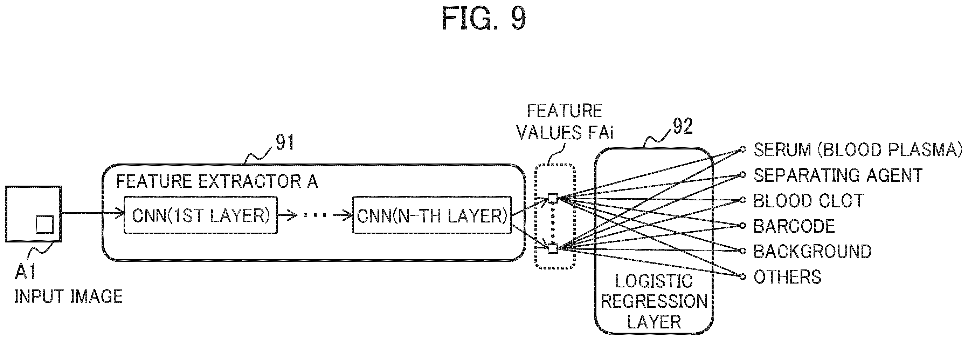

By way of example, Fig. 9 illustrates an example of obtention of the identification result. An input image A1 is input to a feature extractor A91, and feature values FAi are output therefrom. A logistic regression layer 92 identifies a region in the input image A1 on the basis of the feature values FAi. In Fig. 9, CNN represents Convolutional Neural Network.

The inclusion determining unit 13 reads, from the memory 90, a weight w, a filter coefficient wj, and offset values b and bi in Expressions (1) and (2).

For example, using the feature extractor A91, for the upper and lower regions of each of boundary positions and the barcode in the input image A1, the feature values FAi of the inclusions (inclusions such as, e.g., the serum (blood plasma), separating agent, blood clot, and urine) and objects other than the inclusions (such as, e.g., the background and the barcode) are obtained on the basis of Expression (1).

The filter coefficient wj shown in Expression (1) is a coefficient determined by machine learning or the like so as to identify each of the inclusions (such as, e.g., the serum (blood plasma), separating agent, blood clot, and urine) as such and so as to identify each of the objects other than the inclusions (such as, e.g., the background and barcode) as such.

In Expression (1), pj represents a pixel value, bi represents an offset value, m represents the number of filter coefficients, and h represents a non-linear function. Using Expression (1), for each of the regions of the target image from the upper left to the lower right, a calculation result of each of filters is obtained to obtain feature values fi of any filter i. For example, it is assumed that a matrix of the feature values fi obtained by the feature extractor A91 are the feature values FAi of the input image A1.

[Expression 1]

$$fi = h(\sum_{j=1}^{m} (pj \times wj) + bi) \quad \cdots \quad (1)$$

As illustrated in Fig. 9, using the calculated feature values FAi (matrix f) from the feature extractor A91, the logistic regression layer 92 of the identifier calculates a value of the likelihood of each of the inclusions and the objects other than the inclusions to be detected (such as the likelihood of the serum (blood plasma), the likelihood of the separating agent, the likelihood of the blood clot, the likelihood of the background, and the likelihood of the barcode) on the basis of Expression (2) to determine whether or not each of the regions in the input image A1 (e.g., the upper and lower regions of the boundary positions) is the inclusion to be detected (such as, e.g., the serum (blood plasma), separating agent, blood clot, or urine) or the object other than the inclusions to be detected (such as, e.g., the background or the barcode).

In Expression (2), w represents a weight matrix, b represents an offset value, g represents a non-linear function, and y represents an identification result and, by using a known machine learning technology, the weight w and the offset value b are determined in advance by using training images. For example, as the machine learning technology, Convolutional Neural Network may also be used. In addition, by using the weight w and the offset value b which were obtained during leaning, each of the regions of the target image is identified, and an identification result y is calculated.

[Expression 2]

$$y = g(w \times f + b) \quad \cdots \quad (2)$$

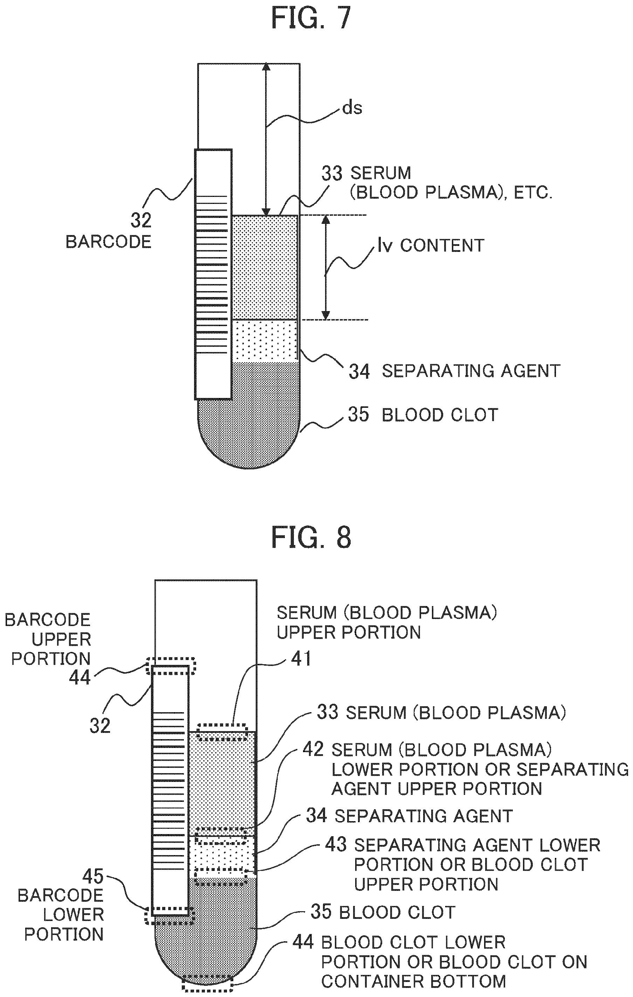

The inclusion determining unit 13 determines what each of the boundaries is on the basis of the identification result y of the upper region and the lower region of each of the boundary positions and the barcode boundaries each detected by the boundary position detecting unit 12. For example, as illustrated in Fig. 8, when the identification result of the upper region is the background and the identification result of the lower region is the barcode 32, the boundary therebetween is determined to be the barcode upper portion 44 (p1 is set to the determination result pr). Meanwhile, when the identification result of the upper region is the barcode 32 and the identification result of the lower region is any of the serum 33, the blood clot 35, the separating agent 34, and the background, the boundary therebetween is determined to be the barcode lower portion 45 (p2 is set to the determination result pr).

Still alternatively, when the identification result of the upper region is the background and the identification result of the lower region is the serum 33, the boundary 41 therebetween is determined to be a serum upper portion (p3 is set to the determination result pr). Yet alternatively, when the identification result of the upper region is the serum 33 and the identification result of the lower region is either of the separating agent 34 and the blood clot 35, the boundary 42 therebetween is determined to be a serum lower portion (p4 is set to the determination result pr). Still alternatively, when the identification result of the upper region is the separating agent 34 and the identification result of the lower region is either of the blood clot 35 and the container bottom, the boundary 43 therebetween is determined to a separating agent lower portion (p5 is set to the determination result pr).

Yet alternatively, when the identification result of the upper region is any of the separating agent 34, the serum 33, and the background and the identification result of the lower region is the blood clot 35, the boundary 43 therebetween is determined to be a blood clot upper portion (p6 is set to the determination result pr). Still alternatively, when the identification result of the upper region is the blood clot 35 and the identification result of the lower region is the separating agent 34, the boundary therebetween (not shown) is determined to be a blood clot lower portion (p7 is set to the determination result pr). Yet alternatively, when the identification result of the upper region of the container bottom is the blood clot 35, the boundary 44 thereof is determined to be the blood clot lower portion (p8 is set to the determination result pr).

Still alternatively, when the identification result of the upper region of the container bottom is the serum 33, the boundary thereof (not shown) is determined to be the serum lower portion (p9 is set to the determination result pr). Yet alternatively, when the identification result of the upper region of the container bottom is the separating agent 34, the boundary thereof (not shown) is determined to be the separating agent lower portion (p10 is set to the determination result pr). Still alternatively, when the identification result of the upper region is any of the serum 33, the background, and the blood clot 35 and the identification result of the lower region is the separating agent 34, the boundary 42 therebetween is determined to be a separating agent upper portion (p11 is set to the determination result pr). For example, in a case of Fig. 5, to the boundary determination results pr, p1, p2, p3, p4, p5, p6, p8, and p11 is set.

The inclusion determining unit 13 excludes boundary candidates which are not the boundary positions and the barcode boundaries each detected by the boundary position detecting unit 12.

The inclusion determining unit 13 stores, in the memory 90, the calculated identification result y and the individual boundary determination results pr.

(iv) Boundary Position Correcting Unit 14

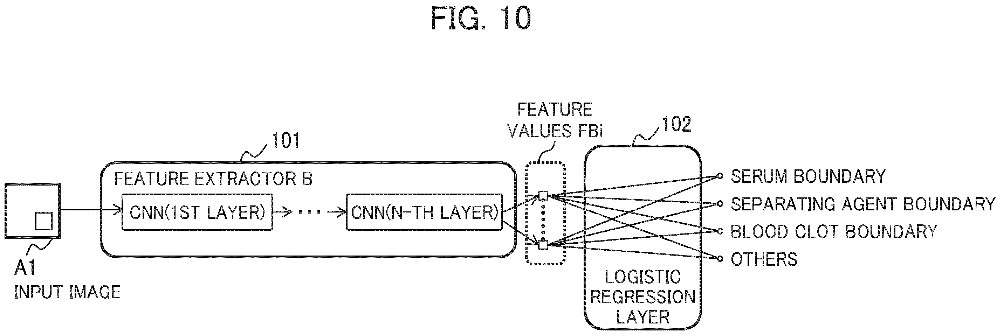

The boundary position correcting unit 14 uses an identifier (including a feature extractor B) formed of a network (such as, e.g., Convolutional Neural Network) that identifies the boundaries of the inclusions (inclusions such as, e.g., the serum (blood plasma), separating agent, blood clot, and urine) in detail to identify the boundaries of each of the inclusions calculated by the inclusion determining unit 13 on the basis of Expressions (1) and (2), while moving on a per pixel basis in an identification area 62 within a retrieval range 61 as illustrated in Fig. 6, and correct each of the boundary positions of the inclusion at a position where the identification result y has a largest value. By way of example, Fig. 10 illustrates an example of obtention of the identification result. The input image A1 is input to a feature extractor B101, and feature values FBi are output. A logistic regression layer 102 identifies a boundary in the input image A1 on the basis of the feature values FBi. In Fig. 10, CNN represents Convolutional Neural Network.

The boundary position correcting unit 14 reads, from the memory 90, the weight w, the filter coefficient wj, the offset values b and bi in Expressions (1) and (2) for identifying the boundaries of each of the inclusion, and calculates the identification result y of the likelihood of each of the boundaries. For example, the boundary position correcting unit 14 identifies the serum boundaries, while vertically moving on a per pixel basis in the identification area 62 within the retrieval range 61 around the serum boundaries, and corrects each of the boundary positions of the serum to a position where the identification result y is largest.

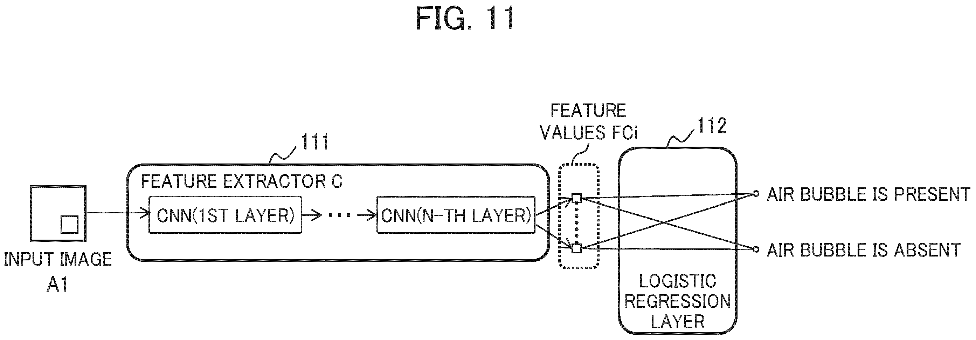

Alternatively, the boundary position correcting unit 14 may also use an identifier (including a feature extractor C) formed of a network (such as, e.g., Convolutional Neural Network) that identifies an air bubble 63 for the boundary of the inclusion determined to be the serum upper portion by the inclusion determining unit 13 to identify the presence or absence of the air bubble in the identification area 62 on the basis of Expressions (1) and (2) and correct, when it is identified that there is the air bubble, the boundary position of the serum upper portion to a position of a lowermost end of the air bubble. By way of example, Fig. 11 illustrates an example of obtention of the identification result. The input image A1 is input to a feature extractor C111, and feature values FCi are output therefrom. A logistic regression layer 112 determines the presence or absence of an air bubble in the input image A1 on the basis of the feature values FCi. In Fig. 11, CNN represents Convolutional Neural Network.

The boundary position correction unit 14 reads, from the memory 90, the weight w, the filter coefficient wj, and the offset values b and bi in Expressions (1) and (2) for identifying the presence or absence of an air bubble, and calculates the identification result y of the air bubble 63.

The boundary position correcting unit 14 stores, in the memory 90, each of the corrected boundary positions, the boundary positions of the barcode, the respective identification results thereof, and the identification result of the air bubble.

(v) Content Calculating Unit 15

The content calculating unit 15 uses the upper and lower boundary positions of each of the inclusions that have been corrected by the boundary position correcting unit 14 to calculate a content lv of each of the inclusions as illustrated in Fig. 7. For example, the content calculating unit 15 reads, from the memory 90, content information of a height from the container bottom and an area inside the container at the height, which is included in the information on the container type identified by the container identifying unit 11, and adds up individual contents from a height of the serum upper portion to a height of the serum lower portion to calculate the content lv of the serum 33. In addition, the content calculating unit 15 calculates a distance ds from a position of the container upper portion calculated by the container identifying unit 11 to the boundary of the serum upper portion.

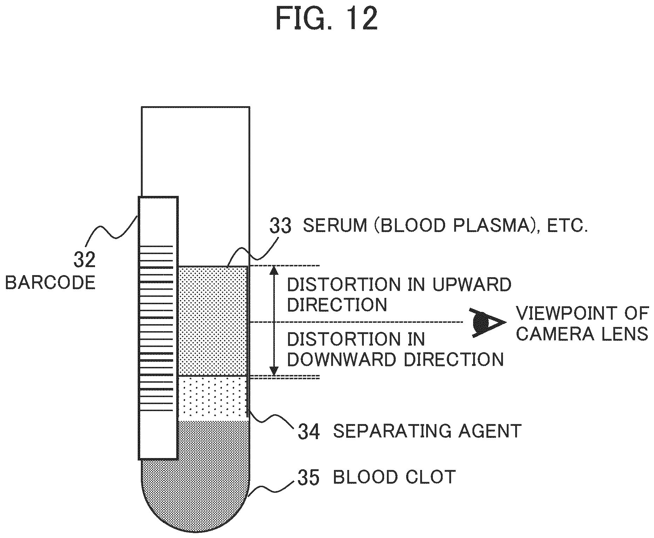

As illustrated in Fig. 12, depending on a viewpoint of a camera lens during photographing or the like, distortion may occur in pixel information as the viewpoint moves in an upward direction or a downward direction. Accordingly, the content calculating unit 15 may also, e.g., correct pixel information (x1, y1) of each of the boundary positions of the serum upper portion and the serum lower portion to (x3, y3) by using Expression (3) to Expression (8), and then calculate the content lv of the serum. Note that, x, y in Expression (3) represent a point including distortion on camera coordinates, x1, y1 therein represent a point including distortion on image coordinates, cx, cy therein represent an image center, and fx, fy therein represent a focal length, k1, k2 in Expression (5) represent a distortion coefficient in a radial direction, p1, p2 in Expression (6) represent a distortion coefficient in a circumferential direction, x2, y2 in Expression (7) represent a point obtained by correcting distortion on the camera coordinates, and x3, y3 in Expression (8) represent a point obtained by correcting distortion on the image coordinates.

[Expression 3]

$$\begin{bmatrix} x \\ y \end{bmatrix} = \begin{bmatrix} (x1 - cx)/fx \\ (y1 - cy)/fy \end{bmatrix} \quad \cdots \quad (3)$$

[Expression 4]

$$r^2 = x^2 + y^2 \quad \cdots \quad (4)$$

[Expression 5]

$$rad = 1 + k1r^2 + k2r^4 \quad \cdots \quad (5)$$

[Expression 6]

$$\begin{bmatrix} dlt\_x \\ dlt\_y \end{bmatrix} = \begin{bmatrix} 2p1xy + p2(r^2 + 2x^2) \\ 2p2xy + p1(r^2 + 2y^2) \end{bmatrix} \quad \cdots \quad (6)$$

[Expression 7]

$$\begin{bmatrix} x2 \\ y2 \end{bmatrix} = \begin{bmatrix} (x - dlt\_x)/rad \\ (y - dlt\_y)/rad \end{bmatrix} \quad \cdots \quad (7)$$

[Expression 8]

$$\begin{bmatrix} x3 \\ y3 \end{bmatrix} = \begin{bmatrix} (x2 \times fx + cx) \\ (y2 \times fy + cy) \end{bmatrix} \quad \cdots \quad (8)$$

The content calculating unit 15 stores, in the memory 90, the content lv and the distance ds each calculated thereby.

(vi) Sample Condition Assessing Unit 16

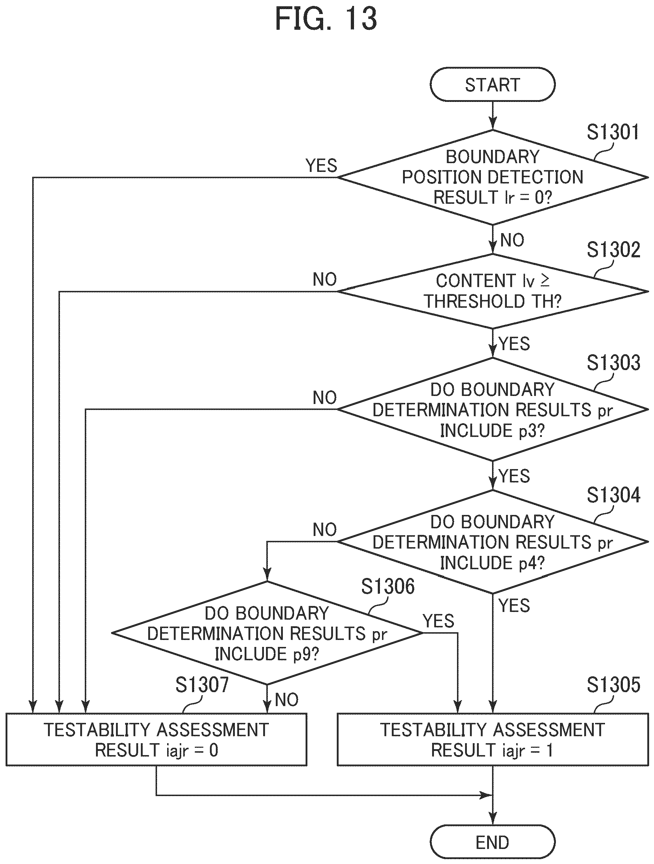

The sample condition assessing unit 16 uses the boundary position detection result lr calculated by the boundary position detecting unit 12, the determination results pr of the individual boundaries calculated by the inclusion determining unit 13, and the content lv calculated by the content calculating unit 15 to assess whether or not the target container is testable. Fig. 13 illustrates a flow chart of the sample condition assessing unit 16.

The sample condition assessing unit 16, for example, assesses that the target container is an empty container when, e.g., the boundary position detection result lr is 0, and sets 0 (untestable) to the testability assessment result, while setting the empty container to a testability assessment reason. When the content lv is less than a threshold TH, the sample condition assessing unit 16 sets 0 (untestable) to the testability assessment result, while setting "Insufficient Content" to the testability assessment reason.

When the boundary determination results pr do not include p3 and p4 or p3 and p9, the sample condition assessing unit 16 sets 0 (untestable) to the testability assessment result, while setting "Serum is absent" to the testability assessment reason. When the boundary position detection result lr is equal to or more than 1, the content lv is equal to or more than the threshold TH, and the boundary determination results pr include p3 and p4 or p3 and p9, the sample condition assessing unit 16 sets 1 (testable) to the testability assessment result iajr, while setting "Serum is present" to the testability assessment reason.

<Processing Procedure in Sample Condition Assessing Unit 16>

(i) Step 1301

The sample condition assessing unit 16 determines whether or not the boundary position detection result lr is 0. When lr is other than 0, processing moves to Step 1302. When lr is 0, the processing moves to Step 1307.

(ii) Step 1302

The sample condition assessing unit 16 assesses whether or not the content lv calculated by the content calculating unit 15 is equal to or more than the threshold TH (e.g., $4\mu l$). When the content lv is equal to or more than the threshold TH, the processing moves to Step 1303. When the content lv is less than the threshold TH, the processing moves to Step 1307.

(iii) Step 1303

The sample condition assessing unit 16 assesses whether or not the determination results pr of the individual boundaries calculated by the inclusion determining unit 13 include p3. When the determination results pr of the individual boundaries include p3, the processing moves to Step 1304. When the determination results pr of the individual boundaries do not include p3, the processing moves to Step 1307.

(iv) Step 1304

The sample condition assessing unit 16 assesses whether or not the determination results pr of the individual boundaries calculated by the inclusion determining unit 13 include p4. When the determination results pr of the individual boundaries include p4, the processing moves to Step 1305. When the determination results pr of the individual boundaries do not include p4, the processing moves to Step 1306.

(v) Step 1305

The sample condition assessing unit 16 sets 1 (testable) to the testability assessment result iajr.

(vi) Step 1306

The sample condition assessing unit 16 assesses whether or not the determination results pr of the individual boundaries calculated by the inclusion determining unit 13 include p9. When the determination results pr of the individual boundaries include p9, the processing moves to Step 1305. When the determination results pr of the individual boundaries do not include p9, the processing moves to Step 1307.

(vii) Step 1307

The sample condition assessing unit 16 sets 0 (untestable) to the testability assessment result iajr.
The sample condition assessing unit 16 stores, in the memory 90, the calculated testability assessment result iajr and the testability assessment reason.

(vii) Output Unit 17

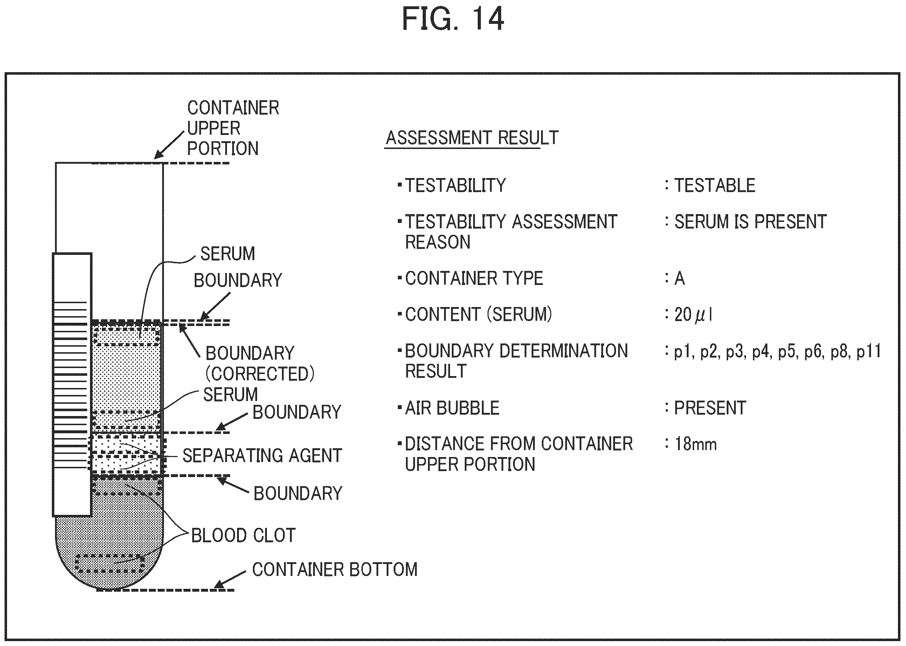

By way of example, the output unit 17 displays, on a GUI (graphical user interface) illustrated in Fig. 14, the container type ct calculated by the container identifying unit 11, a line of each of the boundaries detected by the boundary position detecting unit 12, the identification result of the upper region and the lower region of each of the boundaries and the boundary determination result each resulting from the determination made by the inclusion determining unit 13, a line (e.g.,

boundary (corrected)) of each of the boundaries corrected by the boundary position correcting unit 14, the content lv and the distance ds of each of the inclusions that are calculated by the content calculating unit 15, and the result of the assessment of the testability of the target container made by the sample condition assessing unit 16 and the assessment reason.

Fig. 14 is a diagram illustrating an example of a case where the target container contains the serum, the separating agent, and the blood clot and the boundary position of the serum is corrected. In the example illustrated in Fig. 14, the testability of the target container is "Testable", the testability assessment reason is "Serum is present", the container type is "A", the content (serum) is 20 μl, the boundary determination results are p1, p2, p3, p4, p5, p6, p8, and p11, the identification result of the air bubble is "Present", the distance from the upper portion of the container is 18 mm, and the upper boundary line of the serum, the corrected upper boundary line of the serum, the lower boundary line of the serum (the upper boundary line of the separating agent), the lower boundary line of the separating agent (upper boundary line of the blood clot), and the boundary line of the container bottom are displayed.

The output unit 17 is not an essential component of the specimen condition assessing device 1 and, in a case where the output unit is included in the sample testing device, the specimen condition assessing device 1 need not hold the output unit 17.

<Processing Procedure of Specimen Condition Assessing Device>

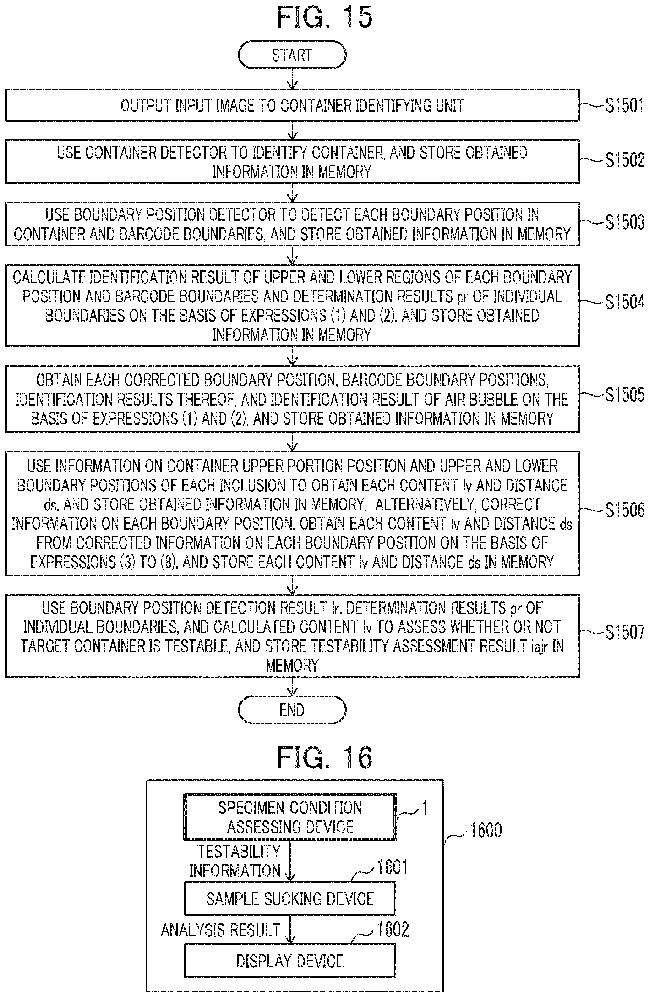

Fig. 15 is a flow chart for illustrating an operation of the specimen condition assessing device 1 according to the embodiment of the present invention. In the following, each of the processing units (such as the input unit 10 and the inclusion determining unit 13) is described as an operating entity, but it may also be read such that the CPU 201 serves as the operating entity and implements each of the processing units as a program.

(i) Step 1501

The input unit 10 receives a target image and outputs the input image to the container identifying unit 11.

(ii) Step 1502

The container identifying unit 11 uses the container detector to detect the container from the input image, further identifies the container type ct, detects respective positions of the container upper portion and the container bottom, and stores the obtained information in the memory 90.

(iii) Step 1503

The boundary position detecting unit 12 uses the boundary position detector to detect each of boundary positions (such as the boundaries of the inclusions) and the barcode boundaries in the container and stores the obtained information in the memory 90.

(iv) Step 1504

The inclusion determining unit 13 calculates the identification result of the upper regions and the lower regions of each of the boundary positions and the barcode boundaries and the determination results pr of the individual boundaries on the basis of Expression (1) and Expression (2), and stores the obtained information in the memory 90.

(v) Step 1505

The boundary position correction unit 14 obtains each of the corrected boundary positions, the boundary positions of the barcode, the respective identification results thereof, and the identification result of the air bubble on the basis of Expression (1) and Expression (2), and stores the obtained information in the memory 90.

(vi) Step 1506

The content calculating unit 15 uses information on the position of the container upper portion and the upper and lower boundary positions of each of the inclusions to obtain the content lv of each of the inclusions and the distance ds from the container upper portion to the serum upper portion, and stores the obtained information in the memory 90. In addition, the content calculating unit 15 corrects the information on each of the boundary positions, obtains the content lv of each of the inclusions and the distance ds from the container upper portion to the serum upper position from the corrected information on each of the boundary positions on the basis of Expression (3) to Expression (8), and stores each content lv and the distance ds in the memory 90.

(vii) Step 1507

The sample condition assessing unit 16 uses the boundary position detection result lr calculated by the boundary position detecting unit 12, the determination results pr of the individual boundaries calculated by the inclusion determining unit 13, and the content lv calculated by the content calculating unit 15 to assess whether or not the target container is testable, and stores the calculated testability assessment result iajr in the memory 90.

According to the first embodiment, by identifying the container type, detecting each of the boundary positions in the container and the barcode boundary positions, calculating the identification result of the upper and lower regions of each of the boundary positions in the container and the barcode, identifying the periphery of each of the boundary positions in detail to correct the boundary position, calculating the content of the target inclusion in the container, and further assessing the condition of the sample in the container by using the calculated content and the identification result of the peripheral region of each of the boundary positions, it is possible to detect the content of the inclusion in the container from the image with high precision and precisely assess whether or not the target container is testable.

In addition, by detecting the content of the inclusion in the container from the image with high precision, it is possible to reduce an amount of the sample collected from a patient to an extremely small amount.

(2) Second Embodiment

Fig. 16 is a functional block diagram illustrating a configuration of a sample testing device 1600 according to a second embodiment of the present invention. The sample testing device 1600 includes the specimen condition assessing device 1 according to the first embodiment, a sample sucking device 1601, and a display device 1602.

The sample sucking device 1601 has a device that controls, e.g., a sample sampling mechanism including a suction nozzle and uses the sucked sample and a reagent to calculate a result of analyzing testing items related to the sample (such as, e.g., HbA1c, TP, and AST related to biochemical analysis and immunity analysis).

The sample sucking device 1601 receives testability information (such as the testability assessment result iajr) from the specimen condition assessing device 1, and controls the sample sampling mechanism when the testability assessment result iajr is 1 (testable) to suck a portion of the sample and calculate the analysis result of each of the testing items by using the reagent. The display device 1602 displays the analysis result on a display screen. Meanwhile, when the testability assessment result iajr is 0 (untestable), the sample sampling mechanism does not suck the sample, and the display device 1602 displays "Untestable" with respect to the testability on the display screen. In addition, the sample sucking device 1601 receives, from the specimen condition assessing device 1, information on the distance ds from the container upper portion and controls a sucking position of the suction nozzle of the sample sampling mechanism.

As the sample sucking device 1601, a biochemical analytical device, an immunity analytical device, or the like including the sample sampling mechanism may also be used.

According to the second embodiment, it is possible to provide a sample testing device that causes the sample sucking device to control whether or not the sample can be sucked with high precision by using the testability information assessed by the specimen condition assessing device and analyze each of the testing items related to the sample by using the sucked sample and the reagent and causes the display device to display the analysis result of each of the testing items to thereby allow efficient testing to be performed and allow an amount of the sample collected from a patient to be reduced to an extremely small amount.

Each of the embodiments described heretofore can be modified as follows. While the inclusion determining unit 13 and the boundary position correcting unit 14 use filters to determine the plurality of feature values by machine learning, other feature values such as HOG may also be used to achieve the same effect.

While each of the container identifying unit 11 and the boundary position detecting unit 12 uses the YOLO as the container detector or the boundary position detector to detect the container or the boundary position, SSD (Single Shot MultiBox Detector), R-CNN, Fast R-CNN, Faster R-CNN, or the like may also be used to achieve the same effect.

The present invention can also be implemented by a program code of software that implements the functions of the embodiments. In such a case, a storage medium with the program code recorded thereon is provided to a system or a device, and a computer (or a CPU or MPU) in the system or device reads the program code stored in the storage medium. In this case, the program code read from the storage medium implements the functions of the embodiments previously described, and the program code and the storage medium with the program code stored thereon form the present invention. As the storage medium for providing such a program code, for example, a flexible disk, CD-ROM, DVD-ROM, a hard disk, an optical disc, a magneto-optical disk, CD-R, a magnetic tape, a nonvolatile memory card, ROM, or the like is used.

In addition, on the basis of an instruction of the program code, an OS (operating system) operating on the computer or the like may perform a part or the whole of actual processing, and the functions of the embodiments previously described may also be implemented by the processing. Moreover, after the program code read from the storage medium is written to a memory in the computer, the CPU or the like of the computer may perform, on the basis of the instruction of the program code, a part or the whole of actual processing, and the functions of the embodiments previously described may also be implemented by the processing.

Furthermore, the program code of the software that implements the functions of the embodiments may be distributed via a network to be stored in a storage means such as a hard disk or memory in the system or device or in a storage medium such as CD-RW or CD-R and, at the time of use, the computer (or the CPU or MPU) in the system or the device may also read the program code stored in the storage means or the storage medium and execute the program code.

Finally, it is to be appreciated that the process and technology described herein may be implemented by any appropriate combination of components without inherently being related to any specific device. Furthermore, various types of general-purpose devices can be used in accordance with the method described herein. It may be advantageous to build a dedicated device to execute the steps of the method described herein. In addition, various inventions can be formed by combining a plurality of components disclosed in the embodiments as appropriate. For example, some components may be removed from all the components shown in the embodiments. Moreover, the components in different embodiments may also be combined as appropriate. While the present invention has been described with reference to specific examples, such examples are shown not for limiting purposes but for description purposes in all aspects. Those skilled in the art may appreciate that there are a large number of combinations of hardware, software, and firmware that are appropriate for implementing the present invention. For example, the software described herein may be implemented by an assembler or a wide range of programs or script languages such as C/C++, perl, Shell, PHP, or Java (registered trademark).

Furthermore, in the embodiments described above, the control lines and information lines represent those that are considered to be necessary for description purposes, and do not necessarily represent all control lines and information lines that are necessary for a product. It may also be possible that all configurations are mutually connected.

In addition, other implementations of the present invention will become apparent to those having ordinary knowledge in this technical field from the examination of the description and the embodiments of the present invention disclosed herein. The various aspects and/or components of the embodiments described above can be used individually or in any combination thereof.

## Claims

1. A specimen condition assessing device comprising:

   a processor that executes a program for performing image processing on an image of a target specimen container; and
   a memory for storing a result of the image processing,
   wherein the processor performs:

   processing of inputting an image;
   processing of detecting a boundary position from the image;
   processing of determining a type of upper and lower regions of each of the detected boundary position and determining a boundary position of each inclusion; and
   processing of assessing whether or not the target specimen container is testable on the basis of the determined boundary position of each inclusion.

2. The specimen condition assessing device according to claim 1,
   wherein the processor further performs:
   processing of correcting each of the detected boundary position on the basis of the determined type of the upper and lower regions of each of the detected boundary position.

3. The specimen condition assessing device according to claim 1,
   wherein the processor further performs:

   processing of identifying a container from the image to calculate container information;
   processing of selecting a detector according to the container information; and
   processing of determining the type of the upper and lower regions of each of the boundary position detected by using the selected detector and determining the boundary position of each inclusion and

the processor assesses whether or not the target specimen container is testable on the basis of the determined boundary position of each inclusion.

4. The specimen condition assessing device according to claim 1, 2, or 3, wherein the processor further performs:

processing of calculating a content on the basis of the determined boundary position of each inclusion and the processor assesses whether or not the target specimen container is testable on the basis of the calculated content.

5. The specimen condition assessing device according to claim 2, wherein the processor further performs:

processing of identifying the container from the image and calculating container information; and processing of calculating a content on the basis of the container information and the determined boundary position of each inclusion and the processor assesses whether or not the target specimen container is testable on the basis of the calculated content.

6. A specimen condition assessing method that assesses a condition of a target specimen container in an image, the specimen condition assessing method comprising:

causing a processor that executes a program for performing image processing on an input image to input an image of the target specimen container; causing the processor to detect a boundary position from the image; causing the processor to determine a type of the upper and lower regions of each of the detected boundary position and determine a boundary position of each inclusion; and causing the processor to assess whether or not the target specimen container is testable on the basis of the determined boundary position of each inclusion.

7. The specimen condition assessing method according to claim 6, further comprising: causing the processor to correct each of the detected boundary position on the basis of the determined type of the upper and lower regions of each of the detected boundary position.

8. The specimen condition assessing method according to claim 6, further comprising:

causing the processor to identify the container from the image and calculate container information; causing the processor to select a detector according to the container information; causing the processor to determine the type of the upper and lower regions of each boundary position detected by using the selected detector and determine the boundary position of each inclusion; and causing the processor to assess whether or not the target specimen container is testable on the basis of the determined boundary position of each inclusion.

9. The specimen condition assessing method according to claim 6, 7, or 8, further comprising:

causing the processor to calculate a content on the basis of the determined boundary position of each inclusion; and causing the processor to assess whether or not the target specimen container is testable on the basis of the calculated content.

10. The specimen condition assessing method according to claim 7, further comprising:

causing the processor to identify the container from the image and calculate container information; causing the processor to calculate a content on the basis of the container information and the determined boundary position of each inclusion; and causing the processor to assess whether or not the target specimen container is testable on the basis of the calculated content.

11. A specimen testing device comprising:

the specimen condition assessing device according to claim 1, 2, or 3;
a specimen sucking device that controls whether or not to suck a specimen to analyze a testing item of the specimen; and
a display device that displays a result of the analysis,
wherein the specimen sucking device uses information on the testability assessed by the specimen condition assessing device to control whether or not to suck the specimen in the specimen container and uses the sucked specimen and a reagent to analyze the testing item related to the specimen and
the display device displays a result of the analysis of the testing item.

# FIG. 1

IMAGE DATA(ex. DYNAMIC IMAGE DATA)

CONTROL UNIT ~ 19

CONNECTED TO EACH ELEMENT

INPUT UNIT ~ 10

CONTAINER IDENTIFYING UNIT ~ 11

BOUNDARY POSITION DETECTING UNIT ~ 12

INCLUSION DETERMINING UNIT ~ 13

BOUNDARY POSITION CORRECTING UNIT ~ 14

CONTENT CALCULATING UNIT ~ 15

90 ~ MEMORY

SAMPLE CONDITION ASSESSING UNIT ~ 16

OUTPUT UNIT ~ 17

OUTPUT RESULT

1

# FIG. 2

1

207  202

MEMORY

10
INPUT UNIT

14/24
BOUNDARY POSITION CORRECTING UNIT

201
CPU

11/21
CONTAINER IDENTIFYING UNIT

15
CONTENT CALCULATING UNIT

204
OUTPUT DEVICE

12/22
BOUNDARY POSITION DETECTING UNIT

16
SAMPLE CONDITION ASSESSING UNIT

205
INPUT DEVICE

13/23
INCLUSION DETERMINING UNIT

17
OUTPUT UNIT

203
STORAGE DEVICE

# FIG. 3

31 CONTAINER UPPER PORTION

CONTAINER IDENTIFICATION

32 BARCODE

33 SERUM (BLOOD PLASMA), ETC.

CONTAINER LENGTH

34 SEPARATING AGENT

35 BLOOD CLOT

36 CONTAINER BOTTOM

# FIG. 4

44 BARCODE BOUNDARY

BOUNDARY POSITION 41

BOUNDARY POSITION 42

43 BOUNDARY POSITION

BARCODE BOUNDARY 45

# FIG. 5

UPPER PORTION

LOWER PORTION

32

UPPER PORTION

CONTAINER LENGTH

33 SERUM(BLOOD PLASMA), ETC.

UPPER PORTION

34 SEPARATING AGENT

LOWER PORTION

UPPER PORTION

35 BLOOD CLOT

UPPER PORTION

# FIG. 6

61 IDENTIFICATION RETRIEVAL RANGE

62 IDENTIFICATION AREA

32
BARCODE

BOUNDARY POSITION CORRECTION

63 BUBBLE

33 SERUM (BLOOD PLASMA), ETC.

34 SEPARATING AGENT

35 BLOOD CLOT

# FIG. 7

ds

33 SERUM
(BLOOD PLASMA), ETC.

32
BARCODE

lv CONTENT

34 SEPARATING AGENT

35 BLOOD CLOT

# FIG. 8

BARCODE
UPPER
PORTION
44

32

SERUM (BLOOD PLASMA)
UPPER PORTION

41

33 SERUM (BLOOD PLASMA)

42 SERUM (BLOOD PLASMA)
LOWER PORTION OR SEPARATING
AGENT UPPER PORTION

34 SEPARATING AGENT

43 SEPARATING AGENT LOWER
PORTION OR BLOOD CLOT
UPPER PORTION

45
BARCODE
LOWER
PORTION

35 BLOOD CLOT

44 BLOOD CLOT LOWER
PORTION OR BLOOD CLOT ON
CONTAINER BOTTOM

# FIG. 9

# FIG. 10

FEATURE EXTRACTOR B

CNN(1ST LAYER) → ⋯ → CNN(N-TH LAYER)

101

FEATURE VALUES FBi

102

LOGISTIC REGRESSION LAYER

A1
INPUT IMAGE

SERUM BOUNDARY
SEPARATING AGENT BOUNDARY
BLOOD CLOT BOUNDARY
OTHERS

EP 4 545 976 A1

FIG. 11

EP 4 545 976 A1

# FIG. 12

32
BARCODE

33  SERUM (BLOOD PLASMA), ETC.

DISTORTION IN UPWARD DIRECTION

DISTORTION IN DOWNWARD DIRECTION

VIEWPOINT OF CAMERA LENS

34  SEPARATING AGENT

35  BLOOD CLOT

# FIG. 13

START

S1301

BOUNDARY
POSITION DETECTION
RESULT lr = 0?

YES

NO

S1302

CONTENT lv ≥
THRESHOLD TH?

NO

YES

S1303

DO BOUNDARY
DETERMINATION RESULTS pr
INCLUDE p3?

NO

YES

S1304

DO BOUNDARY
DETERMINATION RESULTS pr
INCLUDE p4?

NO

YES

S1306

DO BOUNDARY
DETERMINATION RESULTS pr
INCLUDE p9?

YES

NO

S1307

TESTABILITY ASSESSMENT
RESULT iajr = 0

S1305

TESTABILITY ASSESSMENT
RESULT iajr = 1

END

# FIG. 14

CONTAINER
UPPER
PORTION

SERUM
BOUNDARY

BOUNDARY
(CORRECTED)

SERUM
BOUNDARY

SEPARATING AGENT

BOUNDARY

BLOOD CLOT

CONTAINER BOTTOM

ASSESSMENT RESULT

| | |
|---|---|
| ·TESTABILITY | : TESTABLE |
| ·TESTABILITY ASSESSMENT REASON | : SERUM IS PRESENT |
| ·CONTAINER TYPE | : A |
| ·CONTENT (SERUM) | : 20 $\mu$ l |
| ·BOUNDARY DETERMINATION RESULT | : p1, p2, p3, p4, p5, p6, p8, p11 |
| ·AIR BUBBLE | : PRESENT |
| ·DISTANCE FROM CONTAINER UPPER PORTION | : 18mm |

EP 4 545 976 A1

# FIG. 15

START

| OUTPUT INPUT IMAGE TO CONTAINER IDENTIFYING UNIT | S1501 |

| USE CONTAINER DETECTOR TO IDENTIFY CONTAINER, AND STORE OBTAINED INFORMATION IN MEMORY | S1502 |

| USE BOUNDARY POSITION DETECTOR TO DETECT EACH BOUNDARY POSITION IN CONTAINER AND BARCODE BOUNDARIES, AND STORE OBTAINED INFORMATION IN MEMORY | S1503 |

| CALCULATE IDENTIFICATION RESULT OF UPPER AND LOWER REGIONS OF EACH BOUNDARY POSITION AND BARCODE BOUNDARIES AND DETERMINATION RESULTS pr OF INDIVIDUAL BOUNDARIES ON THE BASIS OF EXPRESSIONS (1) AND (2), AND STORE OBTAINED INFORMATION IN MEMORY | S1504 |

| OBTAIN EACH CORRECTED BOUNDARY POSITION, BARCODE BOUNDARY POSITIONS, IDENTIFICATION RESULTS THEREOF, AND IDENTIFICATION RESULT OF AIR BUBBLE ON THE BASIS OF EXPRESSIONS (1) AND (2), AND STORE OBTAINED INFORMATION IN MEMORY | S1505 |

| USE INFORMATION ON CONTAINER UPPER PORTION POSITION AND UPPER AND LOWER BOUNDARY POSITIONS OF EACH INCLUSION TO OBTAIN EACH CONTENT lv AND DISTANCE ds, AND STORE OBTAINED INFORMATION IN MEMORY. ALTERNATIVELY, CORRECT INFORMATION ON EACH BOUNDARY POSITION, OBTAIN EACH CONTENT lv AND DISTANCE ds FROM CORRECTED INFORMATION ON EACH BOUNDARY POSITION ON THE BASIS OF EXPRESSIONS (3) TO (8), AND STORE EACH CONTENT lv AND DISTANCE ds IN MEMORY | S1506 |

| USE BOUNDARY POSITION DETECTION RESULT lr, DETERMINATION RESULTS pr OF INDIVIDUAL BOUNDARIES, AND CALCULATED CONTENT lv TO ASSESS WHETHER OR NOT TARGET CONTAINER IS TESTABLE, AND STORE TESTABILITY ASSESSMENT RESULT iajr IN MEMORY | S1507 |

END

# FIG. 16

SPECIMEN CONDITION ASSESSING DEVICE ~1

~1600

TESTABILITY INFORMATION

SAMPLE SUCKING DEVICE ~1601

ANALYSIS RESULT

DISPLAY DEVICE ~1602

## INTERNATIONAL SEARCH REPORT

| | International application No. |
| --- | --- |
| | **PCT/JP2023/021066** |

**A.   CLASSIFICATION OF SUBJECT MATTER**

*G01N 35/02*(2006.01)i; *G06T 7/00*(2017.01)i; *G06T 7/12*(2017.01)i; *G01N 33/48*(2006.01)i; *G01N 21/88*(2006.01)i
FI:   G01N35/02 C; G01N21/88 J; G01N33/48 H; G06T7/00 612; G06T7/12

According to International Patent Classification (IPC) or to both national classification and IPC

**B.    FIELDS SEARCHED**

Minimum documentation searched (classification system followed by classification symbols)

G01N35/02; G06T7/00; G06T7/12; G01N33/48; G01N21/88

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2021-167733 A (HITACHI HIGH-TECH CORP.) 21 October 2021 (2021-10-21) | 1-11 |
| A | JP 2016-223914 A (HITACHI HIGH-TECHNOLOGIES CORP.) 28 December 2016 (2016-12-28) | 1-11 |
| A | JP 2021-107809 A (F. HOFFMANN-LA ROCHE AG) 29 July 2021 (2021-07-29) | 1-11 |
| A | JP 2020-527712 A (SIEMENS HEALTHCARE DIAGNOSTICS INC.) 10 September 2020 (2020-09-10) | 1-11 |

☐ Further documents are listed in the continuation of Box C.        ☑ See patent family annex.

| | |
| --- | --- |
| *     Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| **11 July 2023** | **25 July 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2023/021066**

| Patent document cited in search report | | | Publication date (day/month/year) | Patent family member(s) | | | Publication date (day/month/year) |
|---|---|---|---|---|---|---|---|
| JP | 2021-167733 | A | 21 October 2021 | WO | 2021/205737 | A1 | |
| | | | | EP | 4134677 | A1 | |
| | | | | CN | 115280158 | A | |
| JP | 2016-223914 | A | 28 December 2016 | (Family: none) | | | |
| JP | 2021-107809 | A | 29 July 2021 | US | 2021/0172725 | A1 | |
| | | | | EP | 3835737 | A1 | |
| | | | | CN | 112948765 | A | |
| JP | 2020-527712 | A | 10 September 2020 | US | 2020/0166531 | A1 | |
| | | | | WO | 2019/018313 | A1 | |
| | | | | CN | 110914667 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022101278 A
- JP HEI101998232228 A
- JP HEI091997133687 A
- JP 2005265813 A